# EUROPÄISCHE PATENTANMELDUNG

(11) **EP 0 876 798 A2**
(43) Veröffentlichungstag der Anmeldung: **11.11.1998**
(21) Anmeldenummer: 98107979.1
(22) Anmeldetag: 30.04.1998
(51) Int. Cl.: A61B 17/82

(54) **Carclage-Spannvorrichtung für die Osteosynthese von Knochenfrakturen**

(30) Priorität: 05.05.1997 DE 19718903
(71) Anmelder: Otto, Rainer, 21614 Buxtehude (DE)
(72) Erfinder: Otto, Rainer, 21614 Buxtehude (DE)
(74) Vertreter: Patentanwälte Hauck, Graalfs, Wehnert, Döring, Siemons

(57) **Zusammenfassung**

Cerclage-Spannvorrichtung für die Osteosynthese von Knochenfrakturen mit
- einem Spannband, das den frakturierten Knochen umschlingt und das mit Schlitzen im Bereich eines ersten Endes und einer eine Öffnung aufweisenden Lasche im Bereich des zweiten Endes zum Hindurchführen des ersten Endes versehen ist, sowie
- einer Spanneinrichtung mit einem Gehäuse, die auf das erste Ende des Spannbandes aufschiebbar und von diesem abziehbar ist, die mit einer drehbar gelagerten Schnecke eines Schneckengetriebes mit den Schlitzen im Spannband in Eingriff bringbar ist und die sich an der Lasche abstützt,
   wobei die Schneckenwelle der Schnecke quer zu ihrer Achse translatorisch beweglich im Gehäuse gelagert ist, um sie wahlweise in oder außer Eingriff (Leerlauf- oder Eingriffsposition) mit den Schlitzen zu bringen, und von Hand betätigbare Sperrmittel die Schnecke in der Eingriffsposition halten.

## Beschreibung

Die Erfindung betrifft eine Cerclage-Spannvorrichtung für die Osteosynthese von Knochenfrakturen gemäß dem Oberbegriff des Patentanspruchs 1.

Bei Knochenbrüchen, die aufgrund der Besonderheit der Bruchformen nur operativ behandelt werden können, stehen dem Chirurgen heute zahlreiche Behandlungsmethoden, wie z.B. die feste Vereinigung der Bruchenden mit Silber- oder Edelstahldraht, das Anschrauben von Metallplatten sowie das Einschlagen von kurzen, rostfreien Stahlnägeln in die Bruchstücke (Schenkelhalsnagelung) oder von langen V2A-Stahlnägeln in den Markraum (Marknagelung nach Küntscher) zur Verfügung.

Dabei haftet all den angedeuteten Verfahren der Nachteil an, daß sie einen relativ hohen zeitlichen Aufwand erfordern und die Metallimplantate nach dem Ausheilungsprozeß wieder entfernt werden müssen.

Des weiteren sind schlauchschellenartige Metallspannbänder im Einsatz, wobei das eine Ende des Bandes mit einer Spanneinrichtung (Spannschloß) fest verbunden ist. Da diese Spannschlösser im Vergleich zur Dicke der Bänder eine große Gehäusehöhe besitzen und nach erfolgter Operation nicht abnehmbar sind, verbleiben sie im Körper und führen aufgrund ihrer Größe zu Reizungen des darüberliegenden Gewebes. Der Heilungsvorgang wird dadurch verzogert, und die Patienten empfinden ein unangenehmes Gefühl beim Betasten der Bruchstelle, insbesondere, wenn Weichteile über dem Knochen nicht in ausreichender Dicke vorhanden sind.

Aus der DE 42 00 757 A1 ist eine entsprechende Vorrichtung aus einem flachbandartigen, mit Querschlitzen versehenen Spannband und einer daran lösbar befestigten Spanneinrichtung bekannt. Dieses abnehmbare Spannschloß ist als Schneckentrieb ausgebildet, der mit Querschlitzen im Spannband in Wirkverbindung steht. Dazu wird zunächst das Spannband um den frakturierten Knochen herumgeschlungen, dann das herumgeschlungene Ende durch eine Lasche am anderen Ende des Spannbands hindurchgeführt, anschließend eine Spanneinrichtung aus Gehäuse und darin dreh- und schwenkbar gelagerter Schnecke auf das freie Ende des Spannbands bis gegen die Lasche geschoben und schließlich die Schnecke so geschwenkt, daß die Schnecke parallel zum Band die Querschlitze im Spannband in Eingriff nimmt. Durch Verdrehen der Schnecke wird das Spannband zusammengezogen, wobei so die für die Fixation der Knochenfraktur erforderliche Vorspannung wie bei Metallspannbändern mit nicht abnehmbarem Spannschloß oder bei Rohrschellen eingestellt werden kann. Nachdem die erforderliche Vorspannung erreicht ist, wird die Spanneinrichtung zusammen mit dem Ende des Spannbandes um die Lasche nach oben gebogen. Das Band ist auf diese Weise in seiner Umfangslänge fixiert, und die Spannvorrichtung kann vom Bandende abgezogen werden. Dazu wird die Schnecke aus dem Eingriff mit den Querschlitzen des Spannbands herausgeschwenkt.

Dieses Ein- und Ausschwenken der Schnecke erfolgt um eine Achse quer über der Bandoberfläche, rechtwinklig zur Erstreckungsrichtung des Spannbands, also im wesentlichen parallel zur Achse des frakturierten Knochens. Die daraus resultierende Schwenkrichtung der Schnecke entspricht also der Biegerichtung des Bands zum Festsetzen seiner Umfangslänge, und es ergibt sich daraus ein großer Schwenkwinkel als notwendiger Betätigungsbewegung zum Handhaben der beschriebenen Spannvorrichtung. Dies ist wegen stets beengter räumlicher Verhältnisse im Operationsfeld des über dem frakturierten Knochen eröffneten Körpers, das zur minimalen Verletzung des operierten Patienten selbstverständlich möglichst klein gehalten werden soll, besonders ungünstig.

Der Erfindung liegt deshalb die Aufgabe zugrunde, eine Cerclage-Spannvorrichtung dahingehend weiter zu verbessern, daß die Spanneinrichtung nach dem Umschlingen, Fixieren und Festsetzen des Spannbands um die Bruchstelle des Knochens abnehmbar ist, somit im Körper nicht belassen werden muß und die Handhabung des Spannbands bzw. der Spanneinrichtung mit möglichst kleinem Zugangs- und Betätigungsraum auskommt.

Diese Aufgabe wird durch eine Spannvorrichtung mit den im Anspruch 1 angegebenen Merkmalen gelöst.

Die erfindungsgemäße Spannvorrichtung zeichnet sich dadurch aus, daß eine Schnecke einer Spanneinrichtung translatorisch in oder außer Eingriff mit Schlitzen in einem Spannband zu bringen ist, indem eine Schneckenwelle quer zu ihrer Achse translatorisch beweglich in einem Gehäuse der Spanneinrichtung gelagert ist. Sperrmittel halten die Schnecke in der Eingriffsposition und sind von Hand betätigbar. In der Eingriffsposition kann ein Lagerabschnitt der Schneckenwelle in ein Lager des Gehäuses einrückbar sein, indem die Schneckenwelle in ihrer Achsrichtung betätigt wird. Vorzugsweise ist die Einrückrichtung des Schneckenwelle mit der Zugrichtung des gespannten Spannbandes an der Schnecke gleichgerichtet, so daß die Zugkraft des gespannten Spannbandes die Schneckenwelle in der Eingriffsposition eingerückt hält. Durch das translatorische Bewegen der Schneckenwelle in bzw. aus dem Eingriff mit den Schlitzen des Spannbandes wird ein nur vorteilhaft kleiner Betätigungsraum der Spanneinrichtung in der Operationswunde am Patienten erreicht. Das Halten der Schnecke in der Eingriffsposition durch die Sperrmittel erleichtert dem Chirurgen die Handhabung der Spanneinrichtung zusätzlich.

Vorteilhafterweise kann eine Feder so zwischen Gehäuse und Schneckenwelle angeordnet sein, daß die Federkraft die Schnecke in eine Leerlaufposition vorspannt, also in die Position, in der sie sich außer Eingriff mit den Schlitzen im Spannband befindet. Dies bewirkt, daß die Schneckenwelle aus dem Eingriff mit den Querschlitzen gehalten wird, wenn sie nicht bewußt in die Eingriffsposition gebracht wurde und durch die Sperrmittel dort gehalten wird. Besonders zum Aufschieben und Abziehen der Spanneinrichtung erleichtert dies die Handhabung beträchtlich.

Ein Drehhandgriff kann zur Betätigung an der Schneckenwelle angebracht sein. Er kann auch abziehbar sein, um die Spanneinrichtungen an mehreren Spannbändern nacheinander zu betätigen und die Spannung mehrerer Spannbänder aufeinander abzustimmen, wenn mehrere Spannbänder zum Versorgen einer Fraktur notwendig sind. Der Drehhandgriff kann mittels einer Vierkant-Zapfenverbindung an der Schneckenwelle abziehbar angebracht sein. Alternativ kann die Schneckenwelle stirnseitig an einem zugänglichen Ende auch einen Schlitz, einen Kreuzschlitz oder einen Innensechskant aufweisen, um anstelle des abziehbaren Drehhandgriffs ein entsprechendes Drehwerkzeug zu verwenden.

Die erfindungsgemäße Cerclage-Spannvorrichtung kann auch zum Befestigen einer Platte auf dem frakturierten Knochen verwendet werden, die sich in Längsrichtung des Knochens erstreckt und die Fraktur mechanisch überbrückt. Dazu wird die Platte auf die Knochenoberfläche gelegt und von einer Anzahl Spannbänder umschlungen. Mittels der erfindungsgemäßen Cerclage-Spannvorrichtung können die Spannbänder in Abstimmung aufeinander und fein dosiert gespannt werden. Erfindungsgemäß hat die Platte in ihrer Oberfläche, die ihrer Auflagefläche auf dem Knochen gegenüberliegt, Ausnehmungen, deren Kontur im wesentlichen einem Abdruck der Unterseite der den Knochen und die Platte umschlingenden Spannbänder im Bereich der Lasche mit hindurchgeführtem ersten Ende und mit aufgeschobener Spanneinrichtung entspricht. Jedes umschlingende Spannband kommt in einer so gestalteten Ausnehmung formschlüssig zu liegen und wird durch diesen Formschluß daran gehindert, in Knochenachsrichtung zu verrutschen oder sich zu verdrehen. Jedes Spannband ist so im Bereich seiner dicksten Stelle, nämlich der Lasche, in die Ausnehmung der Platte eingesenkt und nimmt dadurch zusätzlich vorteilhaft weniger Raum ein.

Ausführungsbeispiele der Erfindung werden im folgenden anhand der beigefügten Zeichnungen beschrieben.
- Fig. 1: zeigt eine perspektivische Darstellung einer erfindungsgemäßen Cerclage-Spannvorrichtung an einem frakturierten Knochen mit abgenommenem Drehhandgriff.
- Fig. 2: zeigt eine teilweise geschnittene Seitenansicht der Spanneinrichtung der Cerclage-Spannvorrichtung in Fig. 1 in Eingriffsposition.
- Fig. 3: zeigt eine teilweise geschnittene Seitenansicht der Spanneinrichtung der Cerclage-Spannvorrichtung in Fig. 1 in Leerlaufposition.
- Fig. 4: eine perspektivische Darstellung einer weiteren Ausführungsform einer erfindungsgemäßen Cerclage-Spannvorrichtung mit geöffnetem Spannband.
- Fig. 5: zeigt eine teilweise geschnittene Seitenansicht der Spanneinrichtung der Cerclage-Spannvorrichtung in Fig. 4 in Leerlaufposition.
- Fig. 6a bis e: zeigen Schnitte durch die Spanneinrichtung in Fig. 5 entlang den Linien III (Fig. 6a), IV (Fig. 6b), V (Fig. 6c), VI (Fig. 6d) bzw. VII (Fig. 6e).
- Fig. 7: zeigt eine perspektivische Darstellung einer weiteren Ausführungsform einer erfindungsgemäßen Cerclage-Spannvorrichtung mit abgenommenem Drehhandgriff.
- Fig. 8: zeigt eine teilweise geschnittene Seitenansicht der Spanneinrichtung der Cerclage-Spannvorrichtung aus Fig. 7 mit angesetztem Drehhandgriff.
- Fig. 9a: zeigt eine Draufsicht auf eine erfindungsgemäße Platte zur Osteosynthese.
- Fig. 9b und c: zeigen Schnitte durch die Platte in Fig. 9a entlang den Linien I (Fig. 9b) bzw. II (Fig. 9c).
- Fig. 10: zeigt eine Draufsicht auf ein erfindungsgemäßes Spannband.

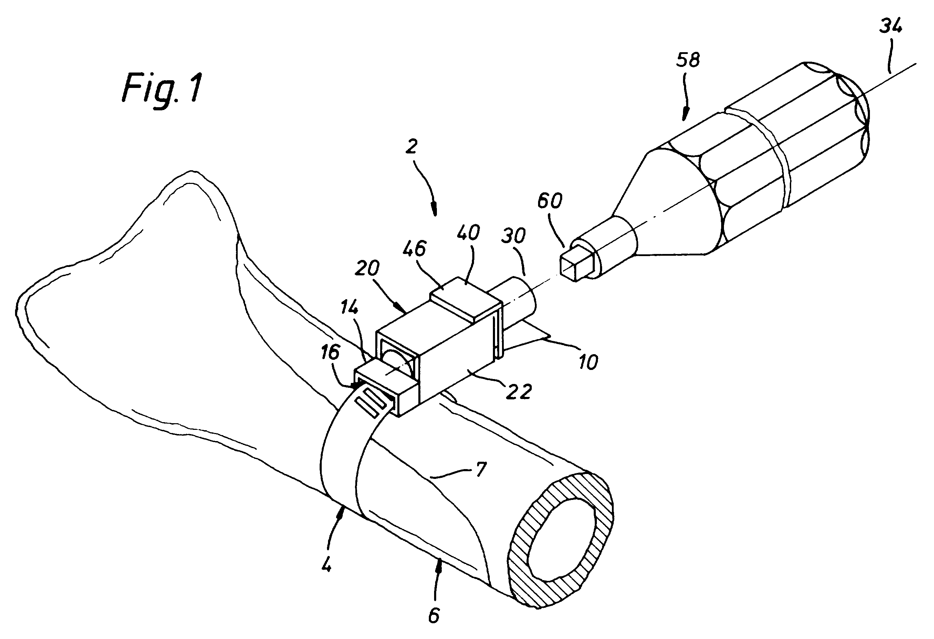

In Fig. 1 ist eine Cerclage-Spannvorrichtung 2 abgebildet, bei der ein Spannband 4 um einen frakturierten Knochen 6 herumgeschlungen ist, um einen Bruchspalt 7 zu komprimieren.

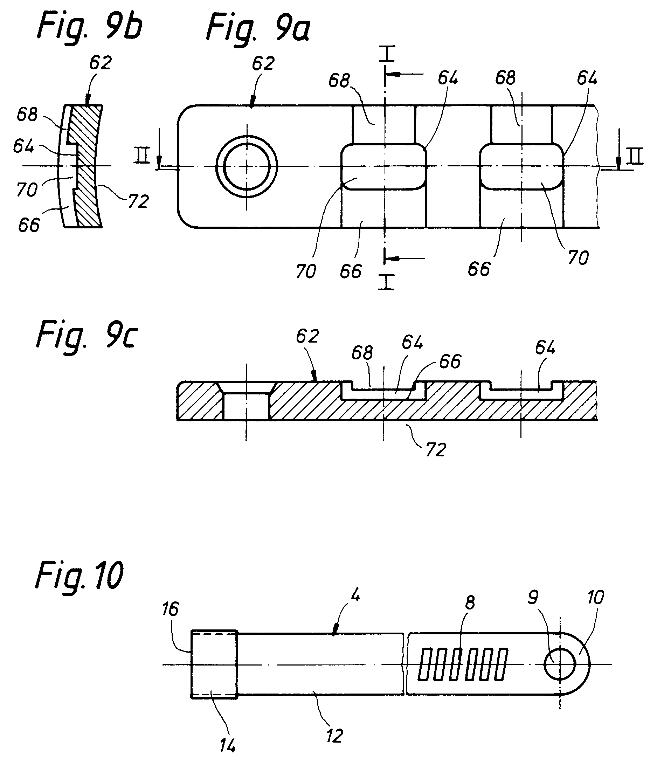

Fig. 10 zeigt das Spannband 4 in flach ausgestreckter Form. Es weist an einem ersten Ende 10 Schlitze 8 auf, die sich parallel zueinander rhomboidförmig quer zur Ausdehnungsrichtung des Bandes erstrecken. An seinem zweiten Ende 12 weist das Spannband 4 eine Lasche 14 auf, durch deren Öffnung 16 das erste Ende 10 hindurchführbar ist, so daß das Spannband 4 zu einem geschlossenen Ring gebogen wird und durch weiteres Hindurchführen des ersten Endes 10 durch die Lasche 14 in der Weite einstellbar ist. Im ersten Ende 10 weist das Spannband 4 zusätzlich ein kreisrundes Loch 9 auf. Im Loch 9 wird das Spannband zum Herumlegen des Spannbands 4 um den Knochen 6 in Fig. 1 mit einer Umführungsnadel gegriffen und manövriert.

In Fig. 1 ist das Spannband 4 um den Knochen 6 herum zu einem geschlossenen Ring gebogen. Das erste Ende 10 des Spannbands erstreckt sich durch die Öffnung 16 in der Lasche 14 sowie durch ein quaderförmiges Gehäuse 22 der Spanneinrichtung 20, das an der Lasche anliegt. Das erste Ende 10 des Spannbands 4 verläuft im Gehäuse 22 entlang der Unterseite des Gehäuses. Eine Schneckenwelle 30, erstreckt sich parallel über der Erstreckungsrichtung des ersten Endes 10 des Spannbandes 4 im Gehäuse 22. Sie ragt an der Seite des Gehäuses 22 aus dem Gehäuse heraus, welche der Seite gegenüberliegt, an der die Lasche 14 anliegt. Ein Drehhandgriff 58 ist mittels einer Vierkant-Zapfenverbindung 60 an das aus dem Gehäuse 22 herausragende Ende der Schneckenwelle 30 formschlüssig ansetzbar und befindet sich in Ansetzposition.

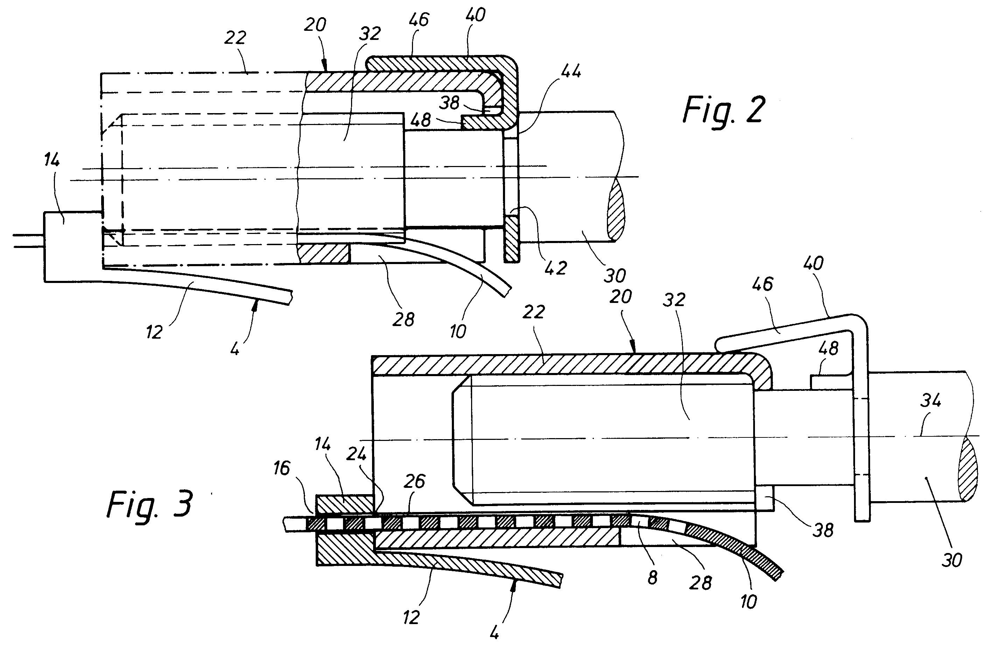

Fig. 2 und 3 zeigen die Spanneinrichtung 20 aus Fig. 1 mit teilweise geschnittenem Gehäuse 22. Die Schneckenwelle 30 ragt an einer Seite aus dem Gehäuse 22 heraus. Auf der Schneckenwelle 30 ist eine Schnecke 32 angeordnet und bildet das Ende der Schneckenwelle 30 im Gehäuse 22. Mit Bezug auf Fig. 3 hat das Gehäuse 22 eine Öffnung 24 in einen Kanal 26, der sich entlang der Unterseite des Gehäuses in eine Ausführöffnung 28 erstreckt. Durch die Öffnung 24 ist das erste Ende 10 des Spannbandes 4 in den Kanal 26 im Gehäuse 22 einführbar. Das erste Ende 10 kann durch die Ausführöffnung 28 aus dem Gehäuse 22 herausragen. Der Kanal 26 ist im Gehäuse 22 an seiner Oberseite offen. Dort kommen die Schlitze 8 im ersten Ende 10 des Spannbands 4 so im Gehäuse 22 zu liegen, daß die Schnecke 32 die Schlitze 8 in Eingriff nehmen kann. Durch Drehung der Schneckenwelle, z.B. mittels des Drehhandgriffs 58 in Fig. 1, wird dann das erste Ende 10 des Spannbands 4 in das Gehäuse hineingezogen. Diese Funktion ist in Fig. 1 deutlich erkennbar: Das Gehäuse 22 stützt sich stirnseitig an der Lasche 14 des Spannbands 4 ab, so daß das erste Ende 10 des Spannbandes 4 durch die Öffnung 16 der Lasche 14 hindurch in das Gehäuse hineingezogen wird, der Umfang des ringförmig gebogenen Spannbandes 4 sich verkleinert und dadurch das Spannband 4 um den Knochen 6 herumgespannt wird.

In Fig. 2 ist die Schneckenwelle bezüglich dem Gehäuse 22 und verglichen mit der Lage in Fig. 3 quer zu ihrer Achse 34 translatorisch nach unten versetzt und in ihrer Achsrichtung in das Gehäuse hineingeschoben. In Fig. 3 ist die Schneckenwelle 30 dementsprechend in Achsrichtung so aus dem Gehäuse 22 herausgerückt, daß die Schnecke 32 noch immer vollständig im Gehäuse verbleibt, und die Schneckenwelle 30 ist quer zu ihrer Achse 34 nach oben verschoben. In diesen beiden Lagen kann die Schnecke die Schlitze 8 im ersten Ende 10 des Spannbands 4 freigeben (Leerlaufposition in Fig. 3) oder in Eingriff nehmen (Eingriffsposition in Fig. 2).

Außerhalb des Gehäuses 22 (Fig. 2) weist die Schneckenwelle 30 eine ringförmige Nut 44 auf, in der sie sich durch eine Bohrung 42 eines Federblech-Streifens 40 hindurcherstreckt. Der Federblechstreifen 40 ist an einem Ende abgewinkelt. Der abgewinkelte Abschnitt 46 erstreckt sich im wesentlichen parallel zur Schneckenwelle 30 und zum Gehäuse 22 und stützt sich federnd auf einer Außenseite des Gehäuses 22 ab (Fig. 1). Durch diese federnde Abstützung wird die Schneckenwelle, wie in Fig. 3 sichtbar, in der Leerlaufposition gehalten.

Der Federblech-Streifen weist eine Nase 48 auf. Die Nase 48 ist parallel zur Schneckenwelle 30 vom Federblech-Streifen 40 in Richtung des Gehäuses 22 abgebogen. Durch ein Langloch 38 ragt die Schneckenwelle 30 aus dem Gehäuse 22 heraus. Im Langloch 38 geführt, ist die Schneckenwelle 30 zwischen der Eingriffs- und der Leerlaufposition verschiebbar. Durch das Einrücken der Schneckenwelle 30 in das Gehäuse 22 hinein, so daß die Schnecke in die Eingriffsposition nach Fig. 2 gerät, rückt auch die Nase 48 in das Langloch 38 ein und sperrt so den Verschiebeweg der Schneckenwelle aus der Eingriffsposition in die Leerlaufposition.

Wenn auf diese Weise die gewünschte Spannung des Spannbands 4 um den Knochen 6 herum eingestellt ist, wird das erste Ende 10 des Spannbands 4 an der Lasche 14 arretiert, z.B. indem das freie Ende mitsamt der Spanneinrichtung 20 um die Lasche 14 herum nach oben gebogen wird. So ist der Umfang des Spannbands 4 um den Knochen 6 festgelegt. Die Spanneinrichtung 20 kann mit dem Drehhandgriff 58 vom ersten Ende 10 des Spannbands 4 abgezogen werden. Mit einem Seitenschneider kann das erste Ende des Spannbands 4 auf die gewünschte Länge gekürzt und um insgesamt 180° um die Lasche 14 herum weitergebogen werden, so daß sich das erste Ende 10 an der Lasche 14 des Spannbands hakenförmig hält.

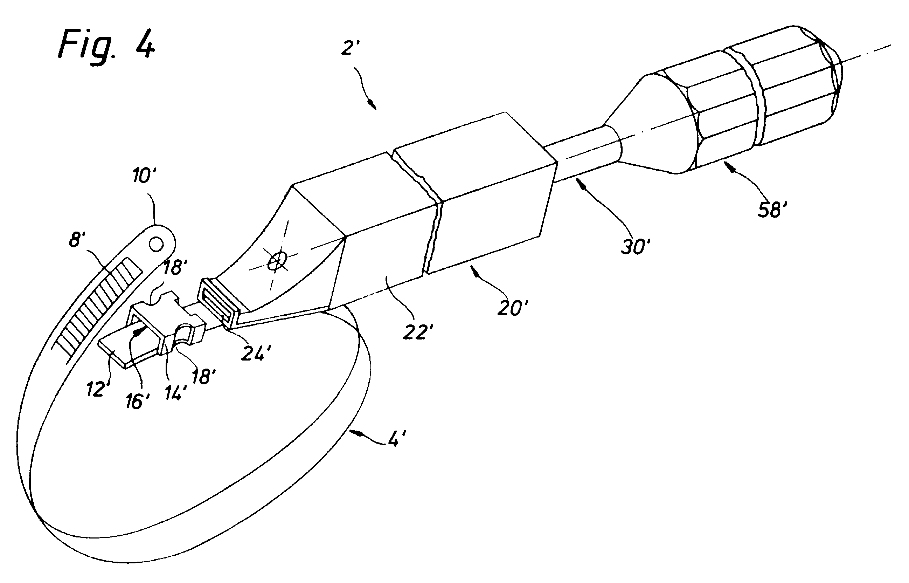

In Fig. 4 weist eine andere Ausführungsform des Spannbands 4' eine Lasche 14' im Bereich seines zweiten Endes 12' auf, die an beiden Seiten jeweils ein seitliches Fenster 18' hat. Wenn das erste Ende 10' des Spannbands 4' durch die Öffnung 16' der Lasche 14' hindurchgeführt ist, kann zum Arretieren des Umfangs des gespannten Spannbands 4' anstelle der eben beschriebenen Handhabung, das erste Ende nach oben zu biegen, durch die seitlichen Fenster 18' das Spannband mit einer Zange so verformt werden, daß das erste Ende 10' in der Lasche 14' formschlüssig festgesetzt ist. Auf diese Weise ist überhaupt keine Schwenkbewegung zur Handhabung der Cerclage-Spannvorrichtung am Patienten nötig.

Zum Spannen des Spannbands 4' weist auch die Spanneinrichtung 20' an der Vorderseite des Gehäuses 22' eine schlitzförmige Öffnung 24' auf, durch die das erste Ende 10' des Spannbands 4' in das Gehäuse 22' einführbar ist. Die Schlitze 8' im ersten Ende 10' des Spannbands 4' kommen im Gehäuse 22' zum Liegen und können dort von einer Schnecke auf einer Schneckenwelle 30' in Eingriff genommen werden, die mittels eines Drehhandgriffs 58' zu betätigen ist.

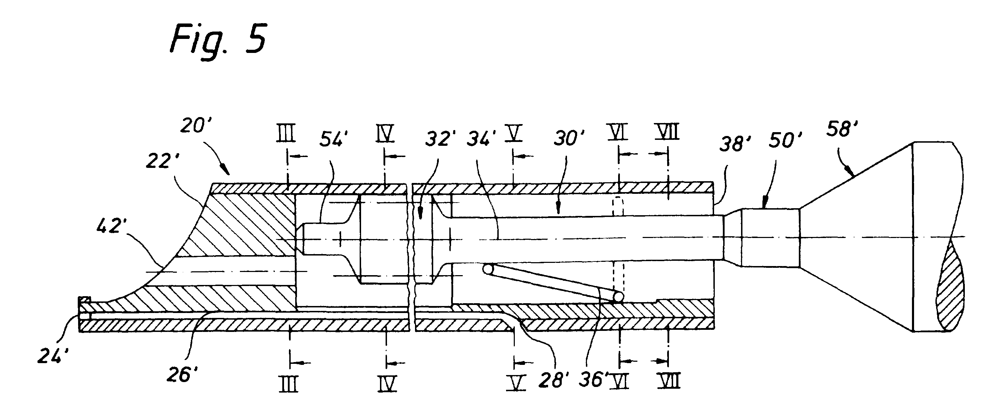

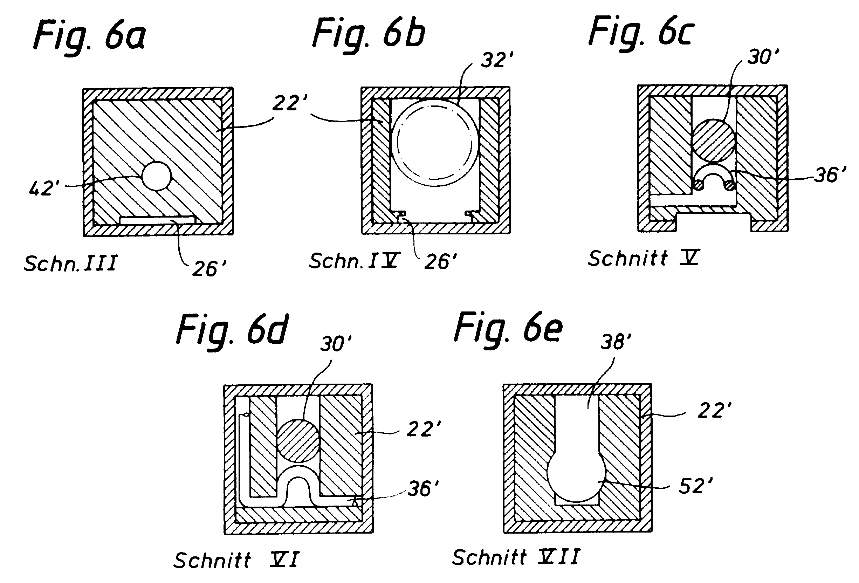

Fig. 5 zeigt die Spanneinrichtung 20' aus Fig. 4 mit geschnittenem Gehäuse 22'. Die schlitzförmige Öffnung 24' an der Stirnseite des Gehäuses mündet in einem Kanal 26', der sich entlang der Unterseite im Gehäuse erstreckt und in einer Ausführöffnung 28' mündet. Durch diesen Kanal 26' wird das erste Ende eines Spannbands hindurchgeführt, so daß die Schlitze im ersten Ende des Spannbands im Bereich des Inneren des Gehäuses 22' zu liegen kommen. Dort ist der Kanal 26' an seiner Oberseite offen. Ebenso wie in der Spanneinrichtung aus Fig. 1 bis 3 ist auch in dieser Ausführungsform eine Schneckenwelle quer zu ihrer Achse 34' translatorisch im Gehäuse 22' verschiebbar angeordnet. Die Schneckenwelle 30' erstreckt sich parallel über der Ausdehnungsrichtung des Kanals 26' für das Spannband und ragt an der Seite des Gehäuses 22', die der Seite mit der schlitzförmigen Öffnung 24' für das Spannband gegenüberliegt, aus einem Langloch 38' heraus. Die Schneckenwelle 30' weist außerhalb des Gehäuses 22' einen Abschnitt mit größerem Durchmesser 50' auf sowie einen Drehhandgriff 58'. Im Gehäuse hat die Schneckenwelle 30' ein zapfenförmiges Ende 54', das direkt an die Schnecke 32' angeformt ist. Das Innere des Gehäuses 22' weist im Bereich des zapfenförmigen Endes 54' der Schneckenwelle 30' eine Lagerbohrung 42' auf. Die Bohrung 42' fluchtet mit dem zapfenförmigen Ende 54' der Schneckenwelle 30', so daß das zapfenförmige Ende 54' in die Bohrung 42' einrückbar ist, wenn die Schnecke in die Eingriffsposition mit dem Schlitzen des Spannbands nach unten verschoben ist. Auf diese Weise ist die Schneckenwelle 30' drehbar in ihrer Eingriffsposition gelagert. Durch die Einrückbewegung rückt auch der Abschnitt mit größerem Durchmesser 50' der Schneckenwelle 30' in eine Aufweitung 52' (Fig. 6e) des Langlochs 38' ein, so daß die Schneckenwelle 30' auch im Bereich des Langlochs 38' drehbar in der Eingriffsposition gelagert und gesperrt ist.

Ein aus Federdraht gebogenes Federelement 36' ist an der Innenseite des Gehäuses 22' fest eingespannt und stützt sich gegen die Schneckenwelle 30' an der dem Kanal 26' zugewandten Seite federnd ab. Wenn die Schneckenwelle 30' aus der Eingriffsposition ausgerückt wird, indem sie um die Länge des Zapfens 54' bzw. des Abschnitts größeren Durchmessers 50' aus dem Gehäuse 22' herausgezogen wird, drückt die Feder 36' die Schneckenwelle quer zu ihrer Achse 34' translatorisch weg vom Kanal 26' des Spannbands und hält die Schnecke außer Eingriff.

Fig. 6a bis e zeigen nebeneinanderliegende Schnitte durch die Spanneinrichtung 20' aus Fig. 5. Anhand dieser Figuren wird die räumliche Ausbildung der beschriebenen Spanneinrichtung 20' aus Fig. 5 deutlich: Fig. 6a zeigt die Lagerbohrung 42' im Inneren des Gehäuses 22' mit quadratischem Querschnitt, in die das zapfenförmige Ende 54' der Schneckenwelle 30' einrückbar ist, sowie die flach rechteckige Kontur des Kanals 26' zur Führung eines Spannbands entlang der Unterseite des Gehäuses. Fig. 6b zeigt einen Querschnitt durch die Schneckenwelle 30' im Bereich der Schnecke 32' im Gehäuse 22' sowie den Kanal 26' im Gehäuse, der in diesem Bereich der Schnecke auf seiner Oberseite zur Schnecke hin offen ist, um einen Eingriff der Schnecke 32' in die Schlitze eines Spannbands zu gestatten. Der Innenraum des Gehäuses 22' ist hoch rechteckig ausgebildet, um eine Translation der Schneckenwelle zwischen Leerlauf- und Eingriffsposition, in der dargestellten Ansicht von oben nach unten, zu erlauben. Fig. 6c und d zeigen im Gehäuse 22' den Verlauf des Federelements 36', das in Fig. 6c an der Schneckenwelle 30' unterseitig anliegt und in Fig. 6d im Gehäuse ohne Freiheitsgrade formschlüssig eingeformt ist. Fig. 6e zeigt das Langloch 38', durch das die Schneckenwelle aus dem Gehäuse 22' herausragt, und die Aufweitung 52' im Langloch, in die der Abschnitt größeren Durchmessers 50' der Schneckenwelle 30' in der Eingriffsposition einrückbar ist.

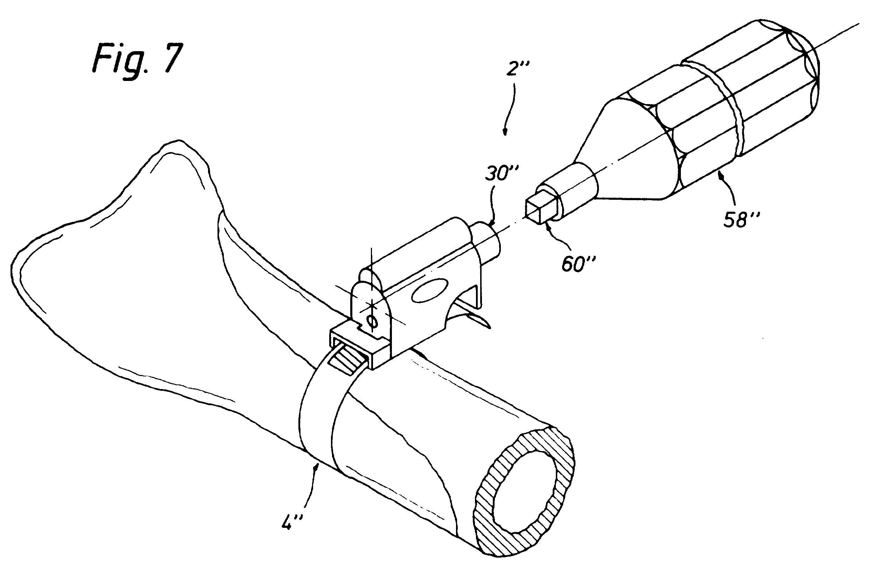

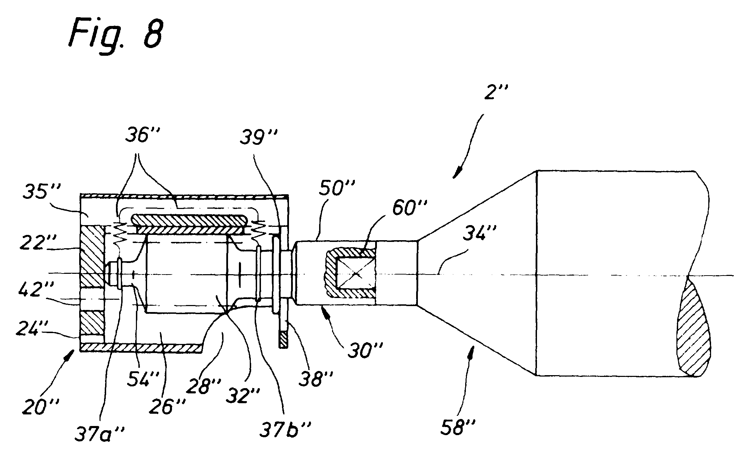

Eine weitere Ausführungsform der Cerclage-Spannvorrichtung in Fig. 7 und 8 unterscheidet sich nur in wenigen Details von der Ausführungsform aus Fig. 4 bis 6: Fig. 7 zeigt, daß der Handgriff 58'' mittels einer Vierkant-Zapfenverbindung 60'' von der Schneckenwelle 30'' abnehmbar ist.

In Fig. 8 sind die Elemente entsprechend Fig. 5 numeriert. Das Gehäuse 22'' ist ebenso wie die Schneckenwelle 30'' kürzer als in Fig. 5 ausgeführt. Die Feder 36'' ist in dieser Ausführungsform eine Spiralzugfeder, die über Drahtringe 37 a,b'' an beiden Seiten der Schnecke 32'' an der Schneckenwelle 30'' angreift. Die Feder 36'' verläuft parallel zur Achse 34'' der Schneckenwelle 30'' in einem dafür vorgesehenen Kanal 35'' im Gehäuse 22''. Der Kanal 35'' für die Feder befindet sich auf der dem Kanal 26'' für ein Spannband gegenüberliegenden Seite der Schneckenwelle 30''. Durch zwei Öffnungen des Kanals 35'' ragen beide Enden der Feder 36'' im Bereich der Ringe 37'' in das Innere des Gehäuses 22'' zur Schneckenwelle 30'' hin. Die Enden der Feder 36'' ziehen die Schneckenwelle 30'' in die Leerlaufposition, also weg vom Kanal 26''. Mittels des Handgriffs 58'' kann die Schneckenwelle 30'' quer zu ihrer Achse 34'' translatorisch zum Kanal 26'' und zum Spannband hin bewegt und dort in Schlitze im ersten Ende eines Spannbands im Kanal 26'' in Eingriff gebracht werden. In dieser Eingriffsposition ist, wie in der Ausführungsform in Fig. 5, ein zapfenförmiges Ende 54'' der Schneckenwelle 30'' in eine Bohrung 42'' im Inneren des Gehäuses 22'' sowie ein Bereich größeren Durchmessers 50'' in eine Aufweitung im Langloch 38'' einrückbar und dadurch in der Eingriffsposition sperrbar.

Ein Sicherungsring 39'' auf der Schneckenwelle 30'' im Bereich der Innenseite des Langlochs 38'' des Gehäuses 22'' sichert die Schneckenwelle 30'' formschlüssig gegen ein völliges Herausziehen aus dem Gehäuse 22''. Ein diesem Sicherungsring 39'' entsprechendes Element weisen die beiden zuvor beschriebenen Ausführungsformen der Cerclage-Spannvorrichtung nicht auf, weil dort die durchmessergrößere Schnecke an der Innenseite des Langlochs die Schneckenwelle gegen vollständiges Herausziehen sichert.

Fig. 9a bis c zeigen eine Platte zur Osteosynthese zur Verwendung in Kombination mit den zuvor beschriebenen Cerclage-Spannvorrichtungen. In der Oberfläche der Platte 62, die der Auflagefläche 72 der Platte 62 auf dem Knochen gegenüberliegt, weist die Platte eine Anzahl von Ausnehmungen 64 auf. Die Kontur der Ausnehmungen 64 entspricht im wesentlichen einem Abdruck der Unterseite eines den Knochen und die Platte umschlingenden Spannbands im Bereich der Lasche mit hindurchgeführtem ersten Ende und mit aufgeschobener Spanneinrichtung. Jede Ausnehmung 64 weist dementsprechend einen tiefen, rechteckigen Bereich 70 in der Mitte der Querrichtung der Platte auf, sowie einen etwas weniger tiefen Bereich 66, der sich mit gleicher Breite wie der tiefe Bereich 70 zum Rand der Platte hin erstreckt und einen noch weniger tiefen Bereich 68, der sich mit geringerer Breite vom tiefen Bereich 70 zum anderen Rand der Platte hin erstreckt.

Jedes Spannband, das die Platte 62 auf einem Knochen aufliegend umschlingt, kann im Bereich einer Ausnehmung 64 so zum Liegen kommen, daß sich die Lasche des Spannbands formschlüssig in den tiefen Bereich 70 der Ausnehmung einformt und das Band sich von der Lasche im Bereich 68 geringer Breite der Ausnehmung fort erstreckt. Diese formschlüssige Einformung des Spannbands im Bereich der Ausnehmung 64 verhindert ein Verrutschen des Spannbands auf der Platte in knochenaxialer Richtung sowie ein Verdrehen. Im Bereich 66 der Ausnehmung ist eine Spanneinrichtung an das dort liegende erste Ende des Spannbands ansetzbar.

## Patentansprüche

1. Cerclage-Spannvorrichtung (2, 2', 2'') für die Osteosynthese von Knochenfrakturen mit
- einem Spannband (4, 4', 4''), das den frakturierten Knochen (6) umschlingt und das mit Schlitzen (8) im Bereich eines ersten Endes (10) und einer eine Öffnung (16) aufweisenden Lasche (14) im Bereich des zweiten Endes (12) zum Hindurchführen des ersten Endes (10) versehen ist, sowie
- einer Spanneinrichtung (20, 20', 20'') mit einem Gehäuse (22, 22', 22''), die auf das erste Ende (10) des Spannbandes (4, 4', 4'') aufschiebbar und von diesem abziehbar ist, die mit einer drehbar gelagerten Schnecke (32, 32', 32'') eines Schneckengetriebes mit den Schlitzen (8) im Spannband (10) in Eingriff bringbar ist und die sich an der Lasche (14) abstützt,
dadurch gekennzeichnet, daß die Schneckenwelle (30, 30', 30'') der Schnecke (32, 32', 32'') quer zu ihrer Achse (34, 34', 34'') translatorisch beweglich im Gehäuse (22, 22', 22'') gelagert ist, um sie wahlweise in oder außer Eingriff (Leerlauf- oder Eingriffsposition) mit den Schlitzen (8) zu bringen, und von Hand betätigbare Sperrmittel die Schnecke (32, 32', 32'') in der Eingriffsposition halten.

2. Cerclage-Spannvorrichtung nach Anspruch 1, dadurch gekennzeichnet, daß in der Eingriffsposition ein Lagerabschnitt (48, 50', 50'', 54', 54'') der Schneckenwelle in ein Lager (38, 52', 38'', 42', 42'') des Gehäuses (22, 22', 22'') durch Betätigung in Achsrichtung der Schneckenwelle (30, 30', 30'') einrückbar ist.

3. Cerclage-Spannvorrichtung nach Anspruch 1 oder 2, dadurch gekennzeichnet, daß die Einrückrichtung der Schneckenwelle (30, 30', 30'') mit der Zugrichtung an der Schnecke (32, 32', 32'') des gespannten Spannbandes (4, 4', 4'') gleichgerichtet ist.

4. Cerclage-Spannvorrichtung nach einem der Ansprüche 1 bis 3, dadurch gekennzeichnet, daß die Schneckenwelle (30, 30', 30'') durch ein Langloch (38, 38', 38'') aus dem Gehäuse (22, 22', 22'') herausragt.

5. Cerclage-Spannvorrichtung nach einem der Ansprüche 1 bis 4, dadurch gekennzeichnet, daß eine Feder (40, 36', 36'') so zwischen Gehäuse (22, 22', 22'') und Schneckenwelle (30, 30', 30'') angeordnet ist, daß die Federkraft die Schnecke (32, 32', 32'') in die Leerlaufposition vorspannt.

6. Cerclage-Spannvorrichtung nach einem der Ansprüche 1 bis 5, dadurch gekennzeichnet, daß an der Schneckenwelle (30) außerhalb des Gehäuses (22) ein Federelement (40) drehbar und axial fest angebracht ist, das sich mit einem Abschnitt (46) an einer Außenseite des Gehäuses (22) abstützt.

7. Cerclage-Spannvorrichtung nach einem der Ansprüche 1 bis 6, dadurch gekennzeichnet, daß die Schneckenwelle (30) außerhalb des Gehäuses (22) eine ringförmige Nut (44) aufweist, mit der sie rotatorisch in einer Bohrung (42) eines Federblech-Streifens (40) gelagert ist, wobei der Federblech-Streifen so abgewinkelt ist, daß sich der abgewinkelte Abschnitt (46) federnd auf einer Außenseite des Gehäuses abstützt.

8. Cerclage-Spannvorrichtung nach Anspruch 7, dadurch gekennzeichnet, daß eine Nase (48), die von dem Federelement (40) zum Langloch (38) hinweisend abgebogen ist, in der Eingriffsposition der Schnecke (32) in das Langloch einrückbar ist und die Schneckenwelle (30) gegen die Bewegung in die Leerlaufposition sperrt.

9. Cerclage-Spannvorrichtung nach einem der Ansprüche 4 bis 7, dadurch gekennzeichnet, daß die Schneckenwelle (30', 30'') im Bereich des Langlochs (38', 38'') einen Abschnitt mit größerem Durchmesser (50', 50'') aufweist und das Langloch eine dem Durchmesser des Abschnitts entsprechende Aufweitung (52', 52''), derart, daß der Abschnitt durch Verschiebung der Schneckenwelle in Achsrichtung in die Aufweitung einrückbar ist.

10. Cerclage-Spannvorrichtung nach einem der Ansprüche 1 bis 9, dadurch gekennzeichnet, daß die Schneckenwelle (30', 30'') im Gehäuse ein zapfenförmiges Ende (54', 54'') aufweist, wobei das Innere des Gehäuses eine Lagerbohrung (42', 42'') aufweist, in die das zapfenförmige Ende der Schneckenwelle in der Eingriffsposition einrückbar ist.

11. Cerclage-Spannvorrichtung nach einem der Ansprüche 1 bis 10, dadurch gekennzeichnet, daß ein Drehhandgriff (58, 58', 58'') an der Schneckenwelle (30, 30', 30'') angebracht ist.

12. Cerclage-Spannvorrichtung nach Anspruch 11, dadurch gekennzeichnet, daß der Drehhandgriff (58, 58'') abziehbar ist.

13. Cerclage-Spannvorrichtung nach Anspruch 12, dadurch gekennzeichnet, daß der Drehhandgriff (58, 58'') mittels einer Vierkant-Zapfenverbindung (60, 60'') an der Schneckenwelle abziehbar angebracht ist.

14. Cerclage-Spannvorrichtung nach einem der Ansprüche 1 bis 10, dadurch gekennzeichnet, daß die Schneckenwelle stirnseitig an einem Ende einen Schlitz, einen Kreuzschlitz oder einen Innensechskant zum Ansatz eines entsprechenden Drehwerkzeugs aufweist.

15. Cerclage-Spannvorrichtung nach einem der Ansprüche 1 bis 14, dadurch gekennzeichnet, daß die Schlitze (8) rhomboidförmig sind.

16. Kombination einer Cerclage-Spannvorrichtung mit einer Platte zur Osteosynthese, dadurch gekennzeichnet, daß die Platte in ihrer Oberfläche, die ihrer Auflagefläche (72) auf dem Knochen gegenüberliegt, mindestens eine Ausnehmung (64) aufweist, deren Kontur im wesentlichen einem Abdruck der Unterseite des den Knochen und die Platte umschlingenden Spannbands im Bereich der Lasche mit hindurchgeführtem ersten Ende und mit aufgeschobener Spanneinrichtung entspricht.
